# FASCICULE DE BREVET EUROPEEN

(11) **EP 0 656 421 B1**
(45) Date de publication et mention de la délivrance du brevet: **28.08.1996**
(21) Numéro de dépôt: 94420339.7
(22) Date de dépôt: 02.12.1994
(51) Int. Cl.: C12Q 1/04, C12Q 1/18

(54) **Milieu nutritif pour la culture de microorganismes**
Nährstoff für Mikroorganismenkultur
Nutrient medium for microorganism culture

(30) Priorité: 02.12.1993 FR 9314687
(43) Date de publication de la demande: 07.06.1995
(73) Titulaire: BIO MERIEUX, F-69280 Marcy l'Etoile (FR)
(72) Inventeur: Jay, Corrine, F-69100 Villeurbanne (FR)
(74) Mandataire: Guerre, Dominique

(56) Documents cités:
- EP-A- 0 019 054
- EP-A- 0 220 379
- EP-A- 0 283 942
- US-A- 4 906 573
- DATABASE WPI Section Ch, Week 8528, Derwent Publications Ltd., London, GB; Class B04, AN 85-168898 & JP-60 098 982 (NAGASE SEIKAGAKU KO) 1 Juin 1985
- J. MILK FOOD TECHNOL., vol.14, 1951 pages 90-91 - 97 M. J. PELCZAR ET AL. 'SYNTHETIC CULTURE MEDIA FOR REFERENCE USE IN DAIRY BACTERIOLOGY'
- Dictionary of Organic Compounds, Fifth Edition, Volume Three, Chapman and Hall, 1982, page 3315

## Description

La présente invention a pour objet un milieu nutritif essentiellement synthétique pour microorganismes. En particulier, l'invention se rapporte à un milieu nutritif pour la culture de microorganismes et son utilisation dans la détermination de l'activité d'une substance active contre lesdits microorganismes.

Par milieu essentiellement synthétique selon l'invention, on entend un milieu dont au moins les constituants indispensables à l'obtention de ce milieu ayant les propriétés décrites plus loin, sont synthétiques.

Les qualités requises pour les milieux nutritifs pour microorganismes sont celles décrites par Ericsson H.M. et al. (J.C. 1977, Antibiotic Sensitivity Testing., Report of an international collaborative study, Munksgaard, Kobenhavn) à savoir :
- permettre la croissance d'une majorité de bactéries cliniquement importantes sans nécessité d'enrichissement complémentaire,
- contenir des éléments de base (peptones) de qualité satisfaisante,
- assurer une reproductibilité des tests de sensibilité quel que soit le lot et/ou le fournisseur,
- ne pas présenter d'antagonisme vis-à-vis des antibiotiques,
- ne pas être sujet à d'importantes variations de pH,
- être isotonique pour des bactéries et supporter l'addition de sang sans provoquer d'hémolyse.

Le milieu actuellement considéré comme milieu de référence par l'ensemble des spécialistes est le milieu Mueller-Hinton gélosé qui satisfait relativement bien toutes ces exigences. Cependant, différents problèmes sont rencontrés qui sont la conséquence de son caractère complexe, le milieu Mueller-Hinton étant constitué d'infusion de viande de boeuf, d'hydrolysat de caséine et d'amidon. Notamment, en ce qui concerne l'étude des antibiotiques, un milieu complexe ne permet pas de maîtriser la concentration des constituants connus, tels que les ions bivalents, la thymidine, le PBA (acide paraaminobenzoïque), l'acide folique, pour optimiser leur activité, ce qui rend fastidieuse, voire impossible, toute tentative visant à corréler une différence d'activité d'un antibiotique avec une différence de composition. Par ailleurs, non seulement la composition du-milieu Mueller-Hinton peut varier d'un lot à l'autre en provenance d'un même fournisseur, mais une importante différence a été mise en évidence lors des changements de fournisseur. Cette variabilité de composition entraîne un manque de reproductibilité de la croissance bactérienne et de l'activité des antibiotiques. Ces problèmes se trouvent accrus avec la forme liquide de ce milieu, qui de plus n'est pas parfaitement adaptée à une utilisation sur un automate, en raison de ses médiocres qualités optiques, du fait qu'il contient des constituants peu solubles comme l'amidon.

Pour résoudre les problèmes de reproductibilité, des milieux dits synthétiques ont été proposés, c'est-à-dire des milieux qui sont essentiellement dépourvus de composants naturels. Par ailleurs, ces milieux lorsqu'ils sont sous forme liquide présentent une qualité optique suffisante pour permettre une mesure fiable et reproductible sur des automates.

Cependant, d'une façon générale, les milieux synthétiques décrits pour l'antibiogramme ne sont présentés que pour quelques espèces, souvent celles ayant les exigences nutritives les plus faibles, et leurs qualités nutritives sont inférieures à celles du milieu Mueller-Hinton de référence (voir par exemple les articles de Hoeprich P.D. et al., Synthetic medium for susceptibility testing, anti-microbial agents and chemotherapy (1970) ; Dougherty P.F. et al., Chemically defined medium for susceptibility testing of antimicrobial agents, Antimicrobial agents and chemotherapy, 10 (6) 2. 923-925 (1976), Lawrence R.M. et al., Totally synthetic medium for susceptibility testing, Antimicrobial agents and chemotherapy, Vol 13, 3, 394-398 (1978)). Par ailleurs, le brevet EP-0 019 054 décrit un milieu synthétique pour la croissance de microorganismes et son utilisation pour la détermination de l'activité d'une substance thérapeutiquement active contre un microorganisme. La composition de ce milieu fait qu'il n'est applicable qu'à la croissance et à la détermination de la sensibilité aux antibiotiques des germes les plus courants et des bactéries anaérobies. De plus, la détermination de la sensibilité aux antibiotiques ne peut être effectuée qu'après 12 à 24 heures d'incubation.

Il devenait donc nécessaire de disposer d'un milieu synthétique qui présente les qualités nutritives du milieu Mueller-Hinton de référence et qui, de plus, soit adapté pour la croissance et la détermination de la sensibilité aux antibiotiques des bactéries les plus couramment isolées en clinique.

Aussi, la Demanderesse a mis au point un milieu qui répond aux exigences précitées et qui par ailleurs, lorsqu'il est sous forme liquide, permet une lecture de l'antibiogramme très rapide, à savoir après 3 à 5 heures d'incubation pour les entérobactéries et moins de 10 heures pour les Pseudomonas par analyse de la cinétique de croissance.

Le milieu selon l'invention comprend un milieu nutritif pour la culture de microorganismes, caractérisé en ce qu'il comprend 7-70 mg/l de L-alanine, 8-80 mg/l de L-arginine, 5-50 mg/l de L-asparagine, 6-60 mg/l d'acide L-aspartique, 2-20 mg/l de L-cystéine, 4-40 mg/l de L-glutamine, 8-80 mg/l d'acide L-glutamique, 7-70 mg/l de glycine, 2,5-25 mg/l de L-histidine, 6-60 mg/l de L-isoleucine, 9-90 mg/l de L-leucine, 9-90 mg/l de L-lysine, 4-40 mg/l de L-méthionine, 5-50 mg/l de L-phénylalanine, 4-40 mg/l de L-proline, 4-400 mg/l de L-sérine, 5-50 mg/l de L-thréonine, 2-20 mg/l de L-tryptophane, 4-40 mg/1 de L-tyrosine, 8-80 mg/l de L-valine, 0,01-0,2 mg/l de biotine, 0,01-0,3 mg/l de pantothénate de calcium, 0,001-0,01 mg/l d'acide folique, 0,01-0,5 mg/l d'inositol, 0,04-0,2 mg/l de vitamine PP, 0,02-0,3 mg/l de vitamine B6, 0,01-0,1 mg/l de chlorure de thiamine, 0,005-0,05 mg/l d'acide lipoïque, 0,1-5 mg/l de choline, 1-10 mg/l d'oxaloacétate d'éthyle, 0,1-1 mg/l de spermidine, 5-50 mg/l de Tween® 80 [sorbitan mono-9-octadecenoate poly(oxy-1,2-ethanediyl) référencé sous le nombre 7455 dans THE MERCK INDEX (loth Edition)], 11-110 mg/l de nucléosides puriques consistant en l'adénosine et la guanosine, dans un rapport de la première à la seconde compris entre environ 0,8 et 1,2, et 4-40 mg/l de nucléosides pyrimidiques, consistant en la cytidine et l'uridine, dans un rapport de la première à la seconde compris entre 1 et 3, les nucléosides puriques et les nucléosides pyrimidiques étant présents dans un rapport des premiers aux seconds compris entre 1,5 et 2,2, 100-10000 mg/l de glucose, 100-10000 mg/l d'acide malique, 0,02-0,583 mg/l de fer, 49,3-493 mg/l de potassium, 1,2-12 mg/1 de magnésium, 2,7-27,2 mg/l de calcium, 1,18-3,15 g/l de sodium, 1820-5000 mg/l en chlorure, 17,8-178,3 mg/l en ion phosphate, 33,6-336,0 mg/l en ion ammonium, 2000-20000 mg/l d'un tampon acide, et 1000-10000 mg/l d'un tampon basique.

En particulier, le milieu selon l'invention comprend 5-50 mg/l d'adénosine, 3-30 mg/l de cytidine, 6-60 mg/l de guanosine, 1-10 mg/l d'uridine, 0,2-5 mg/l de pyrophosphate ferrique, 100-1000 mg/l de phosphate de potassium dibasique, 10-110 mg/l de chlorure de magnésium, 10-100 mg/l de chlorure de calcium, 100-1000 mg/l de chlorure d'ammonium, 3000-8000 mg/l de chlorure de sodium.

Avantageusement, le milieu de l'invention comprend en outre jusqu'à 0,022 mg/l de molybdène, de préférence sous forme de molybdate d'ammonium, en concentration au plus égale à 0,04 mg/l, jusqu'à 0,010 mg/l de cobalt, de préférence sous forme de chlorure de cobalt, en concentration au plus égale à 0,04 mg/l, jusqu'à 0,027 mg/l de manganèse, de préférence sous forme de chlorure de manganèse, en concentration au plus égale à 0,1 mg/l, jusqu'à 0,007 mg/l de zinc, de préférence sous forme de sulfate de zinc, en concentration au plus égale à 0,03 mg/l, jusqu'à 0,128 mg/l de cuivre,de préférence sous forme de sulfate de cuivre, en concentration au plus égale à 0,5 mg/l, jusqu'à 0,088 mg/l de bore, de préférence sous forme d'acide borique, en concentration au plus égale à 0,5 mg/l, jusqu'à 18,4 mg/l d'ion sulfate, de préférence sous forme de sulfate de potassium, en concentration au plus égale à 100 mg/l, jusqu'à 0,1 mg/l de riboflavine, jusqu'à 0,005 mg/l de cyanocobalamine, jusqu'à 0,1 mg/l de ménadione, jusqu' à 0,5 mg/l d'acide borique et/ou jusqu'à 5 mg/l de glycérol.

Dans la présente invention, "jusqu'à" veut dire à une concentration au plus égale à.

Encore plus avantageusement, le milieu selon l'invention comprend 0,0005-0,005 mg/l de cyanocobalamine, 0,01-0,1 mg/l de ménadione, 0,01-0,1 mg/l de riboflavine, 0,1-5 mg/l de glycérol, 0,0004-0,04 mg/l de molybdate d'ammonium, 0,0004-0,04 mg/l de chlorure de cobalt, 0,001-0,1 mg/l de chlorure de manganèse, 0,0003-0,03 mg/l de sulfate de zinc, 2000-20000 mg/l de tampon MOPS [3-[N-morpholino]propane acide sulfonique, commercialisé par la Société SIGMA sous la référence M(254)], 1000-10000 mg/l de tampon Tris (tris(hydroxyméthyl]aminométhane), commercialisé par SIGMA sous la référence T-1503.

Avantageusement, le milieu de l'invention comprend au moins 65 mg/l de L-alanine, 73 mg/l de L-arginine, 43 mg/l de L-asparagine, 53 mg/l de L-acide aspartique, 16 mg/l de L-cystéine, 37 mg/l de L-glutamine, 73 mg/l de L-acide glutamique, 66 mg/l de glycine, 22 mg/l de L-histidine, 54 mg/l de L-isoleucine, 84 mg/l de L-leucine, 89 mg/l de L-lysine, 33 mg/l de L-méthionine, 44 mg/l de L-phénylalanine, 36 mg/l de L-proline, 320 mg/l de L-sérine, 43 mg/l de L-thréonine, 17 mg/l de L-tryptophane, 36 mg/l de L-tyrosine, 70 mg/l de L-valine, 0,03 mg/l de biotine, 0,03 mg/l de pantothénate de calcium, 0,0008 mg/l de cyanocobalamine, 0,005 mg/l d'acide folique, 0,05 mg/l de ménadione, 0,15 mg/l d'inositol, 0,15 mg/l de vitamine PP, 0,15 mg/l de vitamine B6, 0,03 mg/l de riboflavine, 0,03 mg/l de chlorure de thiamine, 0,01 mg/l d'acide lipoïque, 1,5 mg/l de choline, 2,5 mg/l de glycérol, 5 mg/l d'oxaloacétate d'éthyle, 0,75 mg/l de spermidine, 10 mg/l de Tween® 80, 15 mg/l d'adénosine, 10 mg/l de cytidine, 20 mg/l de guanosine, 6 mg/l d'uridine, 750 mg/l de glucose, 500 mg/l d'acide malique, 0,004 mg/l de molybdate d'ammonium, 0,004 mg/l de chlorure de cobalt, 0,016 mg/l de chlorure de manganèse, 0,003 mg/l de sulfate de zinc, 1,8 mg/l de pyrophosphate ferrique, 0,25 mg/l de sulfate de cuivre, 0,025 mg/l d'acide borique, 50 mg/l de sulfate de potassium, 230 mg/l de phosphate de potassium dibasique, 100 mg/l de chlorure de magnésium, 90 mg/l de chlorure de calcium, 500 mg/l de chlorure d'ammonium, 7500 mg/l de chlorure de sodium, 15000 mg/l de tampon MOPS, 7000 mg/l de tampon Tris.

Dans un mode de réalisation de l'invention, le milieu comprend de plus, au moins un peptide à une concentration comprise entre 50 et 100 mg/l ou un mélange de peptides à une concentration comprise entre 50 et 1000 mg/l, les peptides présentant une longueur comprise entre 4 et 15 acides aminés.

En particulier, le peptide est choisi parmi les peptides présentant les séquences d'acides aminés suivantes: 
Ala-Gly-Ser-Glu (SEQ ID NO: 1),
Glu-Asp-Arg-Pro-Pro-Leu-Phe-Gly-Gln-Gly-Thr-Val (SEQ ID NO: 2),
 et

Ala-Ser-Asp-Ala-Lys-Ala-Tyr-Asp-Thr-Glu-Val (SEQ ID NO: 3).

Selon une formulation avantageuse du milieu de l'invention, ce dernier comprend en outre un extrait de boeuf, dont la concentration est de préférence comprise entre 2 et 10 g/l. L'addition d'un extrait de boeuf permet notamment de favoriser la croissance d'espèces telles que Staphylococcus saprophyticus, Xanthomonas maltophilia et Acinetobacter iwoffi.

Le milieu de l'invention peut indifféremment être préparé sous forme liquide ou gélosée. Lorsqu'il est préparé sous forme gélosée, un agent gélifiant est ajouté au milieu. L'agent gélifiant peut être tout agent gélifiant conventionnel connu tel que de la gélatine, de l'agar, de l'agarose, etc. Cependant, pour conserver la nature essentiellement synthétique du milieu, on choisit de préférence l'agarose à une concentration d'environ 10000 mg/l comme agent gélifiant.

Bien entendu, les composants du milieu tels que décrits ci-dessus peuvent être remplacés par des équivalents sans que cela modifie la nature et les propriétés du milieu de l'invention. Ainsi, les sels d'acides ou de bases mentionnés peuvent être remplacés par une quantité équivalente de base libre ou d'acide libre ou par une quantité équivalente d'un sel avec un acide ou une base différent. De même, une base libre ou un acide libre peut être présent sous la forme d'un sel approprié. Egalement, un composant défini comme un hydrate peut être présent sous une forme anhydre ou être remplacé par un hydrate différent qui aurait les mêmes propriétés, selon des critères connus en soi. De même, un agent émulsifiant et dispersant ayant, selon des caractéristiques connues en soi, des propriétés identiques ou voisines du Tween® 80, pourrait être substitué à celui-ci.

L'invention sera mieux comprise à la lecture de la description détaillée qui va suivre, faite en référence aux figures annexées dans lesquelles les abcisses représentent le temps en minutes et les ordonnées une densité optique à 650 nm sur l'appareil MS2 de la Société ABBOTT.

La figure 1 montre l'effet d'une augmentation de la concentration en sérine dans le milieu de l'invention symbolisé par IST, en comparaison avec la croissance obtenue avec le milieu Mueller-Hinton commercialisé par la Société DIFCO LABORATORIES sous la référence 0757-01-4. La courbe a représente la croissance obtenue avec le milieu IST comprenant une concentration initiale de sérine de 32 mg/l. La courbe b représente la croissance avec le milieu IST pour une concentration en sérine augmentée d'un facteur 10. La courbe c représente la croissance obtenue avec le milieu Mueller-Hinton.

La figure 2 représente l'effet de la concentration en acides aminés du milieu IST sur la croissance de Pseudomonas aeruginosa ; a illustre la croissance obtenue avec la concentration initiale des différents acides aminés de 108 mg/l, et b montre l'effet d'une augmentation de la concentration en acides aminés d'un facteur 3.

La figure 3 montre l'influence de la concentration en chlorure de sodium du milieu IST sur la croissance de Proteus mirabilis ; a, b, c et d illustrent cette influence à des concentrations respectives de NaCl, de 0,3% , 0,5%, 1% et 0,75%.

La figure 4 illustre l'influence de la concentration en chlorure de sodium du milieu IST, sur la croissance de Vibrio parahaemolyticus; a, b, c et d correspondent à des concentrations respectives de NaCl, de 0,3% , 0,5%, 0,75% et 1%.

La figure 5 illustre l'effet de l'acide folique sur la croissance d'Enterococcus faecium. La courbe a représente les résultats obtenus sans acide folique, et la courbe b représente la croissance obtenue avec addition au milieu IST de 0,005 mg/l d'acide folique.

La figure 6 montre l'effet de l'acide lipoïque sur la croissance d'Enterococcus faecalis, la courbe a représentant les résultats obtenus avec un milieu IST dépourvu d'acide lipoïque et la courbe b illustrant la croissance de cette souche après addition au milieu IST d'acide lipoïque à une concentration de 0,01 mg/l.

La figure 7 montre l'effet de la spermidine sur la croissance de Proteus mirabilis. a représente les résultats obtenus avec le milieu IST dépourvu de spermidine et b la croissance obtenue après addition de 0,25 mg/l de spermidine.

La figure 8 illustre l'effet de l'oxaloacétate d'éthyle sur la croissance de Staphylococcus saprophyticus. a représente la croissance obtenue avec un milieu IST dépourvu d'oxalacétate d'éthyle, et b représente les résultats obtenus avec 5 mg/l d'oxalacétate d'éthyle.

La figure 9 représente l'effet de la suppression du Tween® 80 sur la croissance d'Enterococcus faecalis. La courbe a représente la croissance obtenue avec un milieu IST dépourvu de Tween 80 et la courbe b les résultats après addition 10 mg/l de Tween 80.

La figure 10 montre l'effet de l'acide L-malique sur la croissance d'Alcaligenes faecalis. La courbe a représente la croissance obtenue avec le milieu IST dépourvu d'acide L-malique et la courbe b les résultats après addition d'une concentration de 500 mg/l d'acide L-malique.

La figure 11 illustre l'effet de l'acide L-malique sur la croissance de Pseudomonas aeruginosa, la courbe a correspondant à la croissance obtenue avec un milieu IST dépourvu d'acide L-malique et la courbe b après addition d'une concentration de 500 mg/l d'acide L-malique.

Les figures 12 et 13 illustrent la compatibilité à une concentration déterminée de l'acide folique avec l'activité de certains antibiotiques. Elles montrent la croissance d'Enterococcus faecalis respectivement sur le milieu IST de l'invention et le milieu Mueller Hinton sans antibiotique (courbes b) et en présence d'un antibiotique, le triméthoprime à une concentration de 0,25 mg/l (courbes a). Ces figures confirment l'inhibition de la croissance de cette souche, quel que soit le milieu, par une concentration de 0,25 mg/l de triméthoprime.

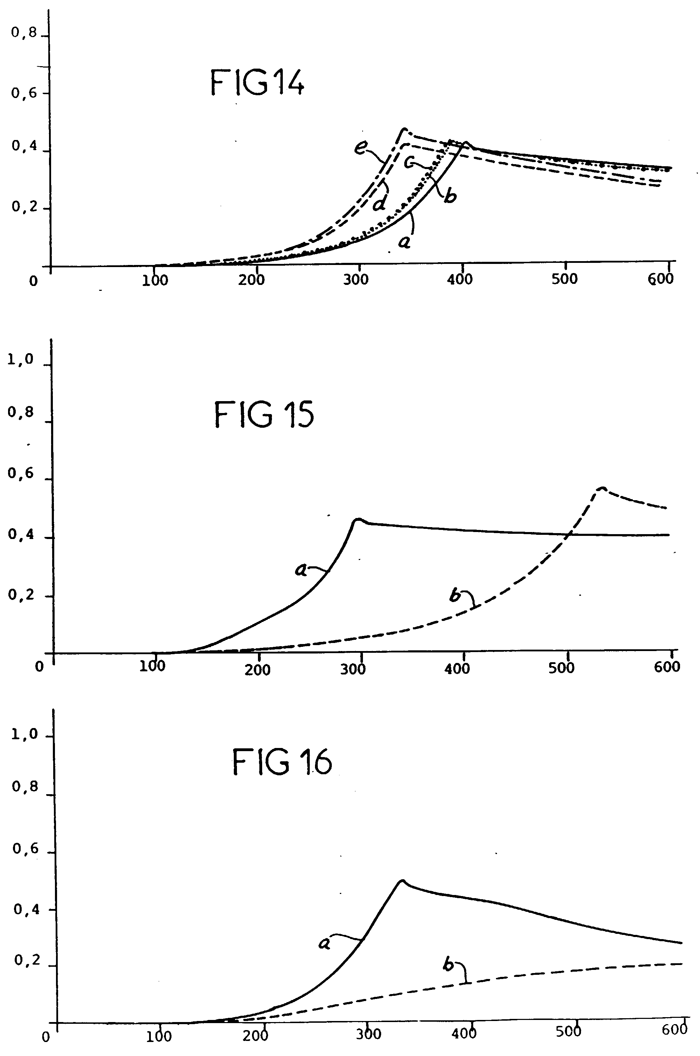

La figure 14 montre l'influence de l'addition du peptide Ala-Gly-Ser-Glu (SEQ ID NO:1) dans le milieu IST de l'invention et la comparaison avec les résultats obtenus par l'ajout d'acides aminés libres constitutifs de ce peptide. La courbe a correspond aux résultats obtenus avec le milieu de l'invention sans addition du peptide précité, ni ajout des acides aminés libres constitutifs de ce peptide. La courbe b illustre les résultats obtenus avec l'addition des acides aminés Ala, Gly, Ser, Glu respectivement à des concentrations de 11, 9, 12,5 et 17,5 mg/l. La courbe c montre les résultats obtenus avec l'addition des acides aminés Ala, Gly, Ser, Glu respectivement à des concentrations de 22, 18, 25 et 35 mg/l. La courbe d correspond à la croissance bactérienne obtenue après addition au milieu de 50 mg/l du peptide. La courbe e illustre les résultats obtenus après addition de 100 mg/l de ce même peptide.

La figure 15 illustre la comparaison entre la croissance d'une souche de S. agalactiae avec le milieu IST de l'invention (courbe a) et le milieu de l'art antérieur (Mueller-Hinton) (courbe b).

La figure 16 établit la comparaison de la croissance d'une souche d'E. faecium avec le milieu de l'invention (courbe a) et le milieu de l'art antérieur (courbe b).

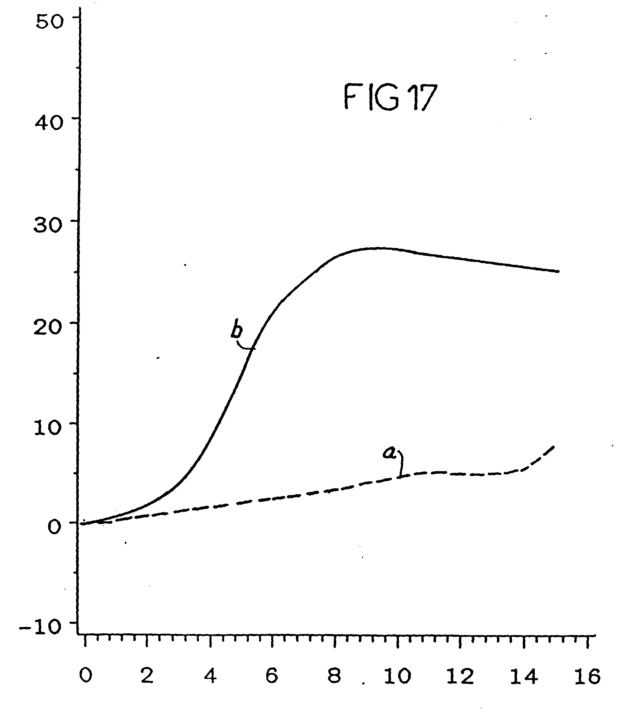

La figure 17 illustre l'influence de l'incorporation d'un extrait de boeuf dans le milieu IST, sur la croissance de Staphylococcus saprophyticus. a représente la croissance obtenue avec un milieu IST dépourvu d'extrait de boeuf, et b représente les résultats obtenus avec 5 g/l d'extrait de boeuf.

### Exemple 1 : composition du milieu

Le milieu selon l'invention comprend des acides aminés, une base minérale, des vitamines et facteurs de croissance, des sources de carbone, des nucléosides et un tampon.

Les 20 acides aminés constitutifs des protéines, tous de la série L, à l'exception de la glycine, sont inclus dans la composition du milieu.

La concentration de chaque acide aminé a été déterminée par référence aux proportions relatives en acides aminés de E.coli, qui est l'espèce bactérienne la mieux connue et la plus souvent isolée en clinique. Les proportions d'acides aminés ont été estimées d'une part par l'analyse chimique d'un hydrolysat de protéine (Neidhart F. C. (1988) Chemical composition of Escherichia coli in Escherichia coli and Salmonella typhimurium, American Society of Microbiology, Washington D.C. vol.1 : 3-6) et d'autre part par l'analyse statistique des codons des protéines fortement exprimées (Manolo G., (1987) Origine et fonction du code génétique chez E. Coli: structuration en banque de données et analyse statistique des séquences nucléotidiques, thèse de Doctorat ès-Sciences, Lyon).

Les résultats obtenus par ces deux approches sont résumés dans le tableau 1 ci-dessous. Les proportions portent sur des concentrations molaires, et sont données en pourcentage d'acides aminés.

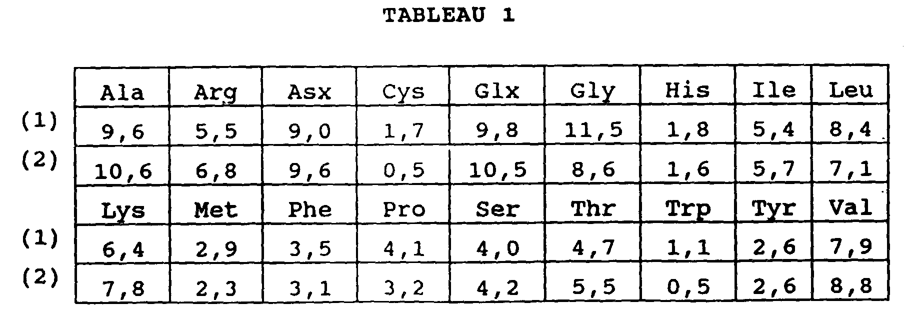

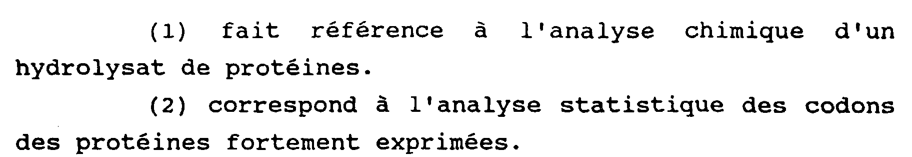

Asx et Glx font respectivement référence à un mélange acide glutamique-glutamine et acide aspartiqueasparagine.

La distinction entre les formes acide et amine de ces acides aminés a été effectuée avec la méthode de Manolo. Les proportions relatives données en pourcentage sont les suivantes : Glu 7,0 %, Gln 3,5 %, Asp 5,5 %, Asn 4,1 %. Les proportions entre les concentrations molaires des différents acides aminés retenues sont celles qui ont été déterminées par la méthode de Neidhart, à l'exception des rapports Glu/Gln et Asp/Asn, qui sont respectivement de 2 et 1,3, déterminés par la méthode de Manolo.

Les proportions en pourcentage des acides aminés du milieu sur la base des concentrations molaires, sont résumées dans le Tableau 2 ci-dessous.

**TABLEAU 2**

| | | | | | | | | | |
|---|---|---|---|---|---|---|---|---|---|
| Ala | Arg | Asn | Asp | Cys | Gln | Glu | Gly | His | Ile |
| 9,6 | 5,5 | 3,8 | 5,2 | 1,7 | 3,3 | 6,5 | 11,5 | 1,8 | 5,4 |
| Leu | Lys | Met | Phe | Pro | Ser | Thr | Trp | Tyr | Val |
| 8,4 | 6,4 | 2,9 | 3,5 | 4,1 | 4,0 | 4,7 | 1,1 | 2,6 | 7,9 |

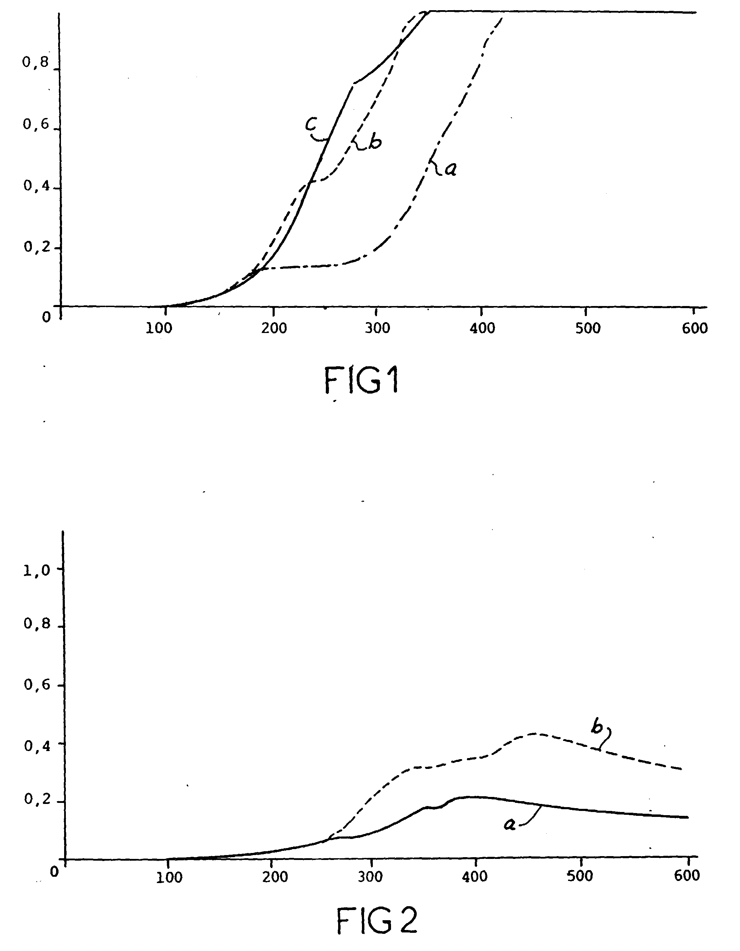

La composition qualitative et quantitative en acides aminés a ensuite été optimisée pour les différentes espèces, afin d'obtenir une croissance conforme à celle obtenue avec le milieu Mueller-Hinton de référence. En particulier, la concentration en sérine a été augmentée pour obtenir une concentration finale voisine de 400 mg/l, avantageusement 320 mg/l, car cet acide aminé est très rapidement assimilé par les Serratia, en particulier les espèces marcescens, liquefaciens et fonticola et les espèces Morganella morganii, Proteus mirabilis et Yersinia enterocolitica. L'amélioration de la croissance est obtenue en augmentant la concentration finale de sérine, notamment pour Serratia marcescens, comme illustré à la figure 1. Comme cela ressort de cette figure, une augmentation d'un facteur 10 de la concentration en sérine permet d'obtenir une croissance bactérienne voisine de celle obtenue avec le milieu Mueller-Hinton. Les concentrations finales en acides aminés ont été définies, après avoir constaté leur importance sur la biomasse finale, en tenant compte de la biomasse obtenue dans le milieu Mueller-Hinton. Cette étude est notamment bien illustrée avec Pseudomonas aeruginosa, dont la croissance et la biomasse finales dépendent fortement de la concentration en acides aminés, comme montré à la figure 2.

La base minérale comprend des ions métalliques et des sels dont le rôle a été décrit par Gottschalk G. (Bacteriol. Metabolism, 2nd Edition, page 3, Spring Verlag., Berlin (1988)). Les ions métalliques sont le bore, le cobalt, le cuivre, le fer, le manganèse, le molybdène, le zinc, qui jouent le rôle de cofacteurs enzymatiques. Les concentrations choisies pour ces ions sont celles définies par Neidhart (Neidhart C. et al., Culture Medium for Enterobacteria, Journal of Bacteriology, Sept. Pages 736-747 (1974)). A l'exception du fer, la suppression des autres oligoéléments n'entraîne pas d'effet significatif sur la croissance des bactéries testées car les dosages réalisés ont montré que ces éléments sont apportés indirectement et en quantité suffisante par les autres constituants du milieu disponibles commercialement. Bien entendu, dans l'hypothèse de l'obtention de produits commerciaux de plus en plus purs, on préfère maintenir un apport contrôlé des ions minéraux afin d'assurer une concentration minimum toujours compatible avec la croissance des bactéries. Au contraire, le fer s'avère être un constituant additionnel indispensable, notamment pour la croissance de certaines espèces. La concentration du fer dans le milieu, qui est relativement élevée, a été optimisée pour satisfaire les exigences de P. aeruginosa et des staphylocoques sans inhiber la croissance des autres espèces. A titre d'exemple, différents sels de fer peuvent être inclus dans le milieu, tels que le pyrophosphate de fer, le chlorure de fer, le citrate de fer et le sulfate de fer, indépendamment ou en combinaison. De préférence, le constituant de l'invention est le sel de pyrophosphate.

Le soufre est essentiellement apporté par l'ion sulfate, qui représente 45 % du soufre présent dans le milieu, mais certaines bactéries, qui ont perdu leur capacité à réduire l'ion sulfate, pourront utiliser d'autres composés réduits présents dans le milieu, notamment la cystéine et la méthionine. De préférence, l'ion sulfate du milieu de l'invention est le sulfate de potassium. La concentration en sulfate choisie est celle définie par Neidhart.

La source principale de potassium (70%) et de phosphore (100%) est apportée par le phosphate de potassium dibasique. La concentration de phosphate a été déterminée afin d'éviter la formation de précipités avec les ions calcium et magnésium affectant la qualité optique du milieu et modifiant les concentrations de ces deux ions. La concentration choisie est celle définie par Neidhart.

L'ion ammonium constitue une source d'azote utilisée par la majorité des bactéries. La concentration en ions ammonium a été définie en tenant compte des concentrations en acides aminés, qui sont également utilisées comme source d'azote par les bactéries. La forme chlorure est préférée, notamment par rapport aux formes phosphate, ceci afin d'éviter un apport supplémentaire d'ions phosphate.

Les ions calcium et magnésium sont les cofacteurs de nombreuses enzymes et sont connus pour modifier la sensibilité à certains antibiotiques, notamment les aminosides vis-à-vis de Pseudomonas (Christe M. et al. (1982) Susceptibilité du Pseudomonas aeruginosa aux aminosides: influence du calcium et du magnésium, Schweizerische Medizinische Wochenschrift, 112, 4, 241). Les teneurs en ions calcium et magnésium du milieu de l'invention correspondent aux normes préconisées pour l'antibiogramme par le NCCLS (National Committee for Clinical Laboratory Standards, 1990, Methods for dilution antimicrobial susceptibility tests for bacteria that grow aerobically. Approved standard publication M7-A2: 10-11 Villanova Pa USA). Les concentrations pondérales en ions calcium et magnésium sont respectivement de 25 et 12,5 mg/l. Les formes chlorure de calcium et chlorure de magnésium sont avantageusement choisies dans les constituants du milieu de l'invention.

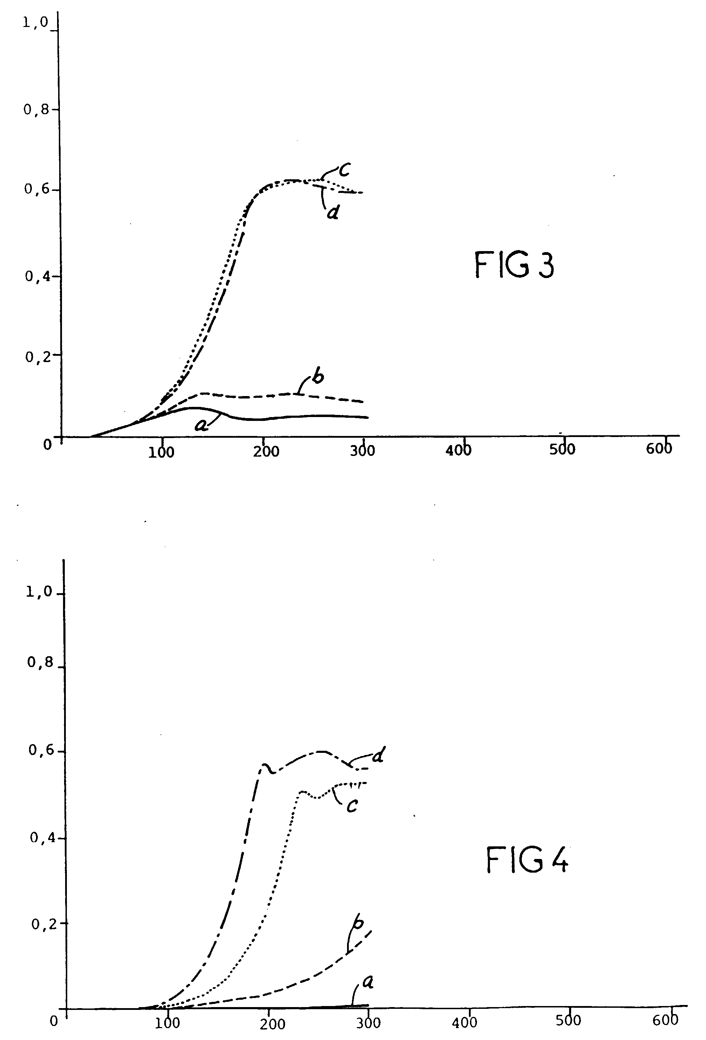

Le chlorure de sodium représente la source principale d'ions sodium et chlorure. La concentration en NaCl a été optimisée en tenant compte des fortes exigences en sodium de Proteus mirabilis et de Vibrio parahaemolyticus, comme montré aux figures 3 et 4.

Les vitamines et facteurs de croissance sont des constituants importants pour les métabolismes des bactéries. Le rôle des vitamines B1, B2, B5, B6, B12, H et PP dans le métabolisme bactérien est précisé dans plusieurs ouvrages, dont le "Manual of Methods for General Bacteriology" de Guirard et Snell (Biochemistry Factors in Growth, chapter 7 (1981)). L'inositol, la choline et le glycérol sont des précurseurs des lipides cellulaires dont les phospholipides membranaires (voir Klig L.S. et al. (1990) Phospholipid biosynthesis in Candida albicans: regulation by the precursors inositol and choline, J. Bact. 178(8): 4407-4414). Les concentrations en acide folique, acide lipoïque, spermidine, oxaloacétate d'éthyle et Tween® 80 ont été particulièrement bien étudiées pour le milieu de l'invention.

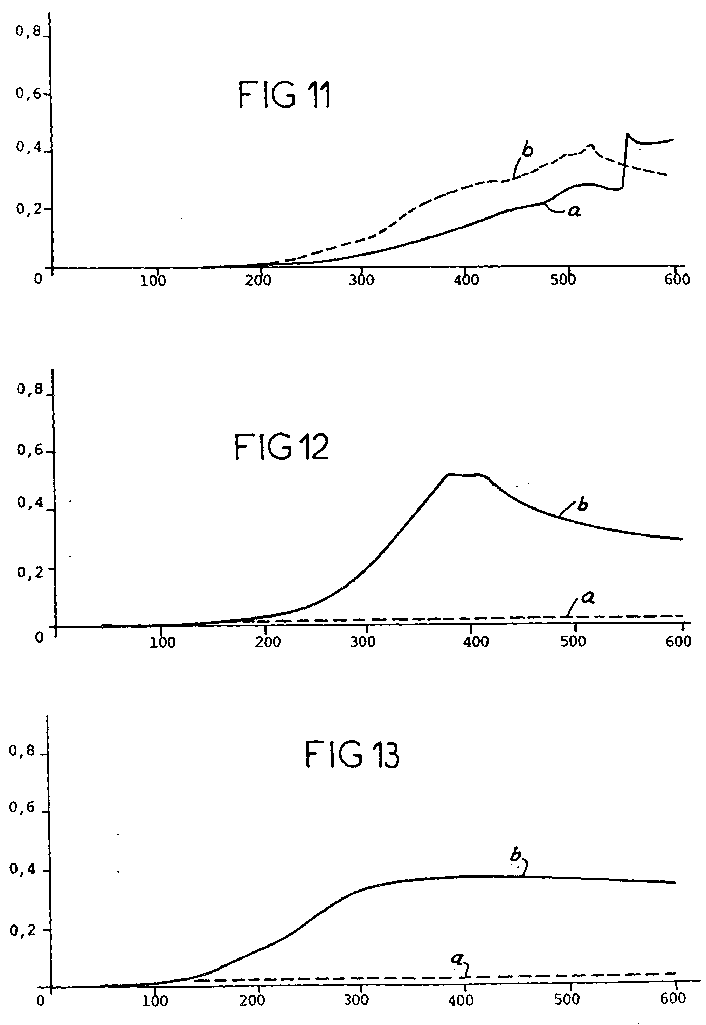

L'acide folique est un coenzyme dont l'importance pour la croissance des entérocoques, notamment pour les différentes souches d'E. faecalis, E. faecium et E. hirae a été démontrée, comme décrit ci-après. Il est par ailleurs connu pour affecter l'activité des antibiotiques de la famille des sulfamides et du triméthoprime (Then R. et al., Mécanisme moléculaire de la bactéricidie; sulfamides et triméthoprime. Bactéricidie 1990, Editions Maloine, Paris). La concentration en acide folique a été déterminée pour satisfaire les exigences des souches testées et pour sa compatibilité avec l'activité des antibiotiques, comme illustré aux figures 12 et 13.

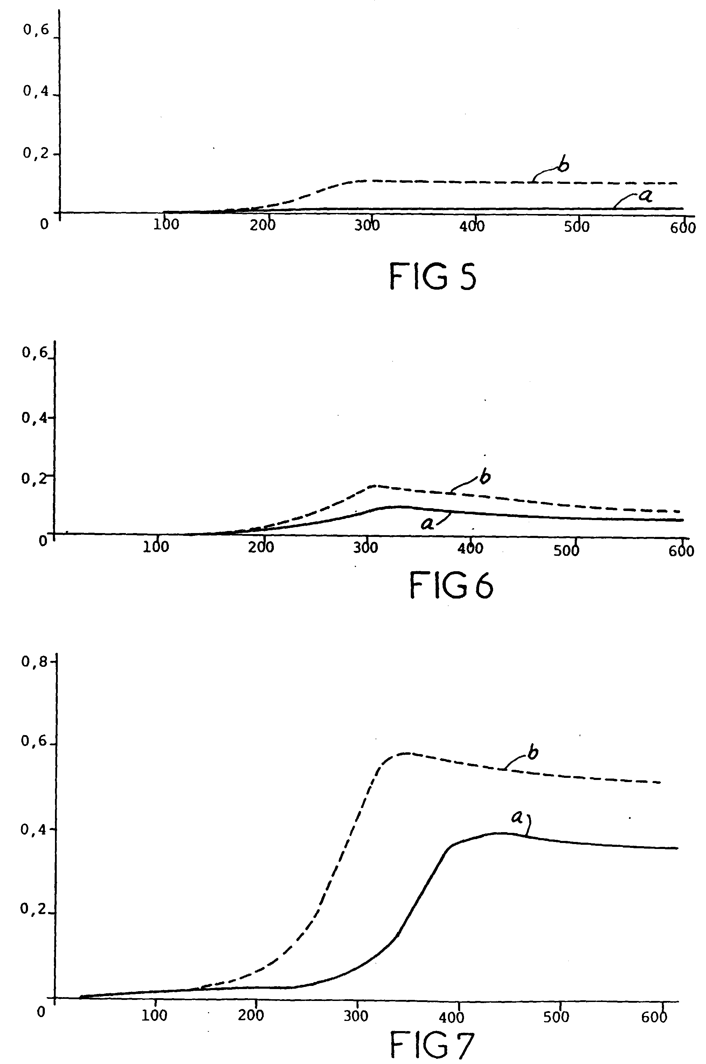

La concentration en acide lipoïque a été déterminée comme la concentration minimum permettant d'améliorer au maximum la croissance des entérocoques et des streptocoques du groupe B. La figure 6 illustre l'effet de l'acide lipoïque sur la croissance d'Enterococcus faecalis.

Les polyamines sont présentes dans les cellules eucaryotes et procaryotes et participent à de nombreuses réactions biologiques dont la synthèse des acides nucléiques et des protéines (voir Tabor C.W. and Tabor H. (1985) Polyamines in Microorganisms, Microbiol. Rev. 49:81-99). La putrescine, la spermine et la spermidine ont été testées. La spermidine a été choisie en fonction des résultats, notamment pour la croissance des Proteus, Morganella, Providencia et Serratia à une concentration préférentielle de 0,75 mg/l. La figure 7 illustre l'effet de la spermidine sur la croissance de Proteus mirabilis.

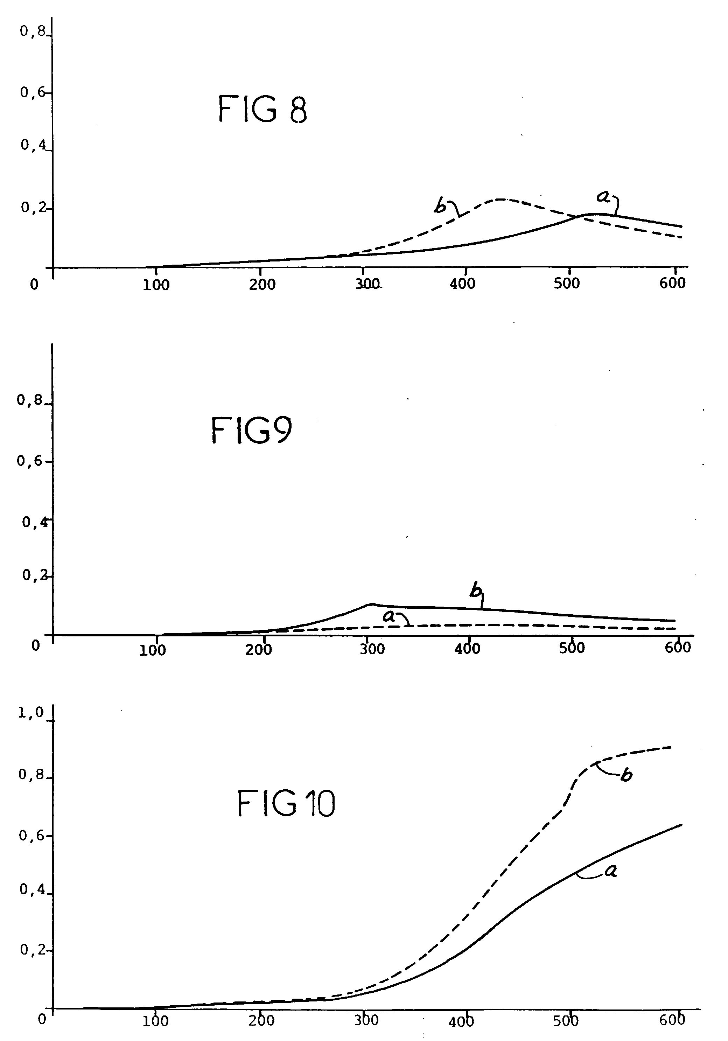

L'oxaloacétate d'éthyle est décrit pour son effet stimulateur sur la croissance de nombreuses espèces (Pelczar M.J. et Brown J.H. (1951) Synthetic culture media for reference use in dairy bacteriology, J. Milk Food Technol., 14: 90-91 et 97). Son influence sur la croissance des souches de staphylocoques a été mise en évidence, comme montré à la figure 8, et la concentration retenue est celle permettant d'améliorer la croissance des staphylocoques sans inhiber celle des autres espèces.

Le Tween® 80 est un agent émulsifiant et dispersant. Il peut remplacer la biotine comme montré par Gretler A.C. et al. (Vitamine nutrition of the staphylococci with special reference to their biotin requirements (1954) J. Bact. 70: 44-49). Il joue également un rôle détoxifiant et protecteur en absorbant les éventuels éléments métabolites toxiques du milieu (voir Klein R.D. et al. (Simplified media for growth of haemophilus influenzae for clinical and normal flora sources. Journal of General Microbiology. 113: 409-411 (1979)). Comme montré à la figure 9, la suppression de Tween® 80 dans le milieu entraîne une diminution de croissance des différentes espèces bactériennes.

Les sources de carbone du milieu de l'invention sont représentées majoritairement par un sucre, de préférence D-glucose et un acide organique, en particulier l'acide L-malique. Le D-glucose a été retenu, compte tenu de son utilisation par un grand nombre d'espèces bactériennes à métabolisme fermentatif ou non (Stanier R.Y. et al. (1966) The aerobic Pseudomonas: a taxonomic study. J. Gen. Microbiol. 43 : 159-271). Sa concentration dans le milieu de l'invention a été déterminée pour atteindre une biomasse proche de celle obtenue avec le milieu Mueller-Hinton de référence. L'acide L-malique est nécessaire à la croissance des espèces non glucidolytiques, telles qu'Alcaligenes faecalis, comme montré à la figure 10, et Pseudomonas acidovorans. Il améliore également la croissance de Pseudomonas aeruginosa qui l'utilise préférentiellement au glucose comme source de carbone, comme montré à la figure 11. Les concentrations respectives en D-glucose et en acide L-malique ont été déterminées pour répondre aux qualités du Mueller-Hinton.

Les bases puriques et pyrimidiques peuvent être apportées sous plusieurs formes, c' est-à-ciire sous forme de précurseurs, de bases libres, de nucléosides ou de nucléotides. Les études sur le milieu de l'invention ont montré que l'apport des deux précurseurs hypoxanthine et uracile ne satisfait pas les exigences en base des entérocoques. Par ailleurs, les nucléotides entraînent une diminution de la croissance de nombreuses espèces bactériennes. Des améliorations significatives ont été obtenues avec les nucléosides, en l'absence de la thymidine compte tenu de son incompatibilité avec l'activité des sulfamides et du triméthoprime. Les proportions entre purines et pyrimidines ont été définies par rapport à celles décrites pour la synthèse de l'ARN par Neidhart (Neidhart et al. (1990) Physiology of the bacterial cell. A molecular approach. Sinauer associates, inc. USA), en tenant compte de l'absence de thymidine, c'est-à-dire purines/pyrimidines dans un rapport compris entre 1,5 et 2,2 et avantageusement de 1,8. Les concentrations finales ont été ensuite optimisées essentiellement pour satisfaire la croissance des staphylocoques et des entérocoques.

Le tampon a été choisi pour répondre aux exigences de qualité optique du milieu et de pH, le pH 7,4 étant adapté à l'antibiogramme et à la croissance des bactéries. La forme acide est apportée par le tampon MOPS, qui a été particulièrement choisi en raison de son pKa de 7,2 (à 20°C) proche du pH du milieu. La concentration du tampon MOPS a été définie de façon à assurer un pouvoir tampon équivalent à celui du milieu Mueller-Hinton sans dépasser la concentration de 80mM, au-delà de laquelle des inhibitions de croissance bactérienne ont été décrites par Neidhart. La forme basique particulièrement retenue est le tampon Tris, qui a été préféré à une base forte comme l'hydroxyde de sodium, afin d'éviter un apport supplémentaire d'ions. La concentration dans le milieu de l'invention a été définie en fonction de celle du tampon MOPS et du pH final souhaité.

La Demanderesse a par ailleurs démontré que l'addition de peptides dans le milieu de l'invention avait un effet stimulateur, notamment pour la croissance des entérocoques. Parmi les peptides testés, seuls ceux présentant une taille inférieure ou égale à 15 acides aminés entraînent une forte amélioration de la croissance. Par ailleurs, la Demanderesse a montré que l'addition des acides aminés libres constitutifs des peptides, apportés en concentration équivalente, ne permet pas de reproduire l'effet stimulateur, comme illustré à la figure 14. En particulier, le tétrapeptide Ala-Gly-Ser-Glu (SEQ ID NO:1) entraîne une amélioration considérable de la croissance de différentes souches d'Enterococcus hirae et d'Enterococcus faecium, d'autres peptides tels que Glu-Asp-Arg-Pro-Pro-Leu-Phe-Gly-GIn-Gly-Thr-Val (SEQ ID NO:2) et Ala-Ser-Asp-Ala-Lys-Ala-Tyr-Asp-Thr-Glu-Val (SEQ ID NO:3) donnant des résultats intéressants pour d'autres souches d'entérocoques.

### Exemple 2: Préparation du milieu de l'invention.

Le milieu selon l'invention peut être préparé, par exemple, à partir de six solutions mères concentrées qui sont obtenues directement à partir des constituants commerciaux en poudre, référencés dans le catalogue de la société SIGMA, dilués avec une quantité requise d'eau distillée. Les solutions sont ensuite stérilisées par filtration et réfrigérées sous forme liquide ou congelées à -20°C. Les solutions mères, qui sont respectivement des solutions de la base minérale (5X concentrée), des oligo-éléments (10 000X concentrée), des acides aminés (3X concentrée), des nucléosides (8X concentrée), des vitamines hydrosolubles (700X concentrée), des vitamines liposolubles (2000X concentrées), sont ensuite mélangées entre elles, puis diluées avec une quantité requise d'eau distillée et additionnées aux glucose, acide L-malique et phosphate de potassium dibasique (constituants commercialisés par la société SIGMA). Si nécessaire le pH est ajusté par addition de soude ou d'acide chlorhydrique pour obtenir une valeur voisine de 7,4. L'ordre dans lequel sont mélangés les différents constituants n'est pas critique.

### Exemple 3 : comparaison des résultats obtenus avec le milieu de l'invention et le milieu Mueller-Hinton de référence.

En vue de la détermination de la sensibilité aux antibiotiques, une suspension de 0,5 MF (Mac Farland) dans de l'eau physiologique a été préparée à partir de colonies bactériennes prélevées sur boites de Pétri, la lecture étant effectuée sur un densitomètre ATB 1550 commercialisé par la société BIOMERIEUX (ATB marque de commerce). La suspension obtenue a respectivement été diluée au 1/100ème dans le milieu de l'invention et le milieu Mueller-Hinton. Des cartes VITEK (VITEK marque de commerce) contenant différents antibiotiques à 10 concentrations ont été remplies sous vide dans un appareil VITEK commercialisé par la société BIOMERIEUX-VITEK. Les cartes ont été incubées pendant 12 heures dans un incubateur/lecteur VITEK commercialisé par la société BIOMERIEUX-VITEK. Les cinétiques de croissance ont été visualisées, interprétées et une CMI équivalente a été déterminée (CMI équivalente: concentration minimale d'inhibition équivalente, c'est-à-dire la plus petite concentration pour laquelle aucune croissance bactérienne n'est détectée par l'appareil).

Les résultats ont été présentés dans les tableaux 3 et 4 ci-après.

IST fait référence au milieu de l'invention et MH signifie milieu Mueller-Hinton.

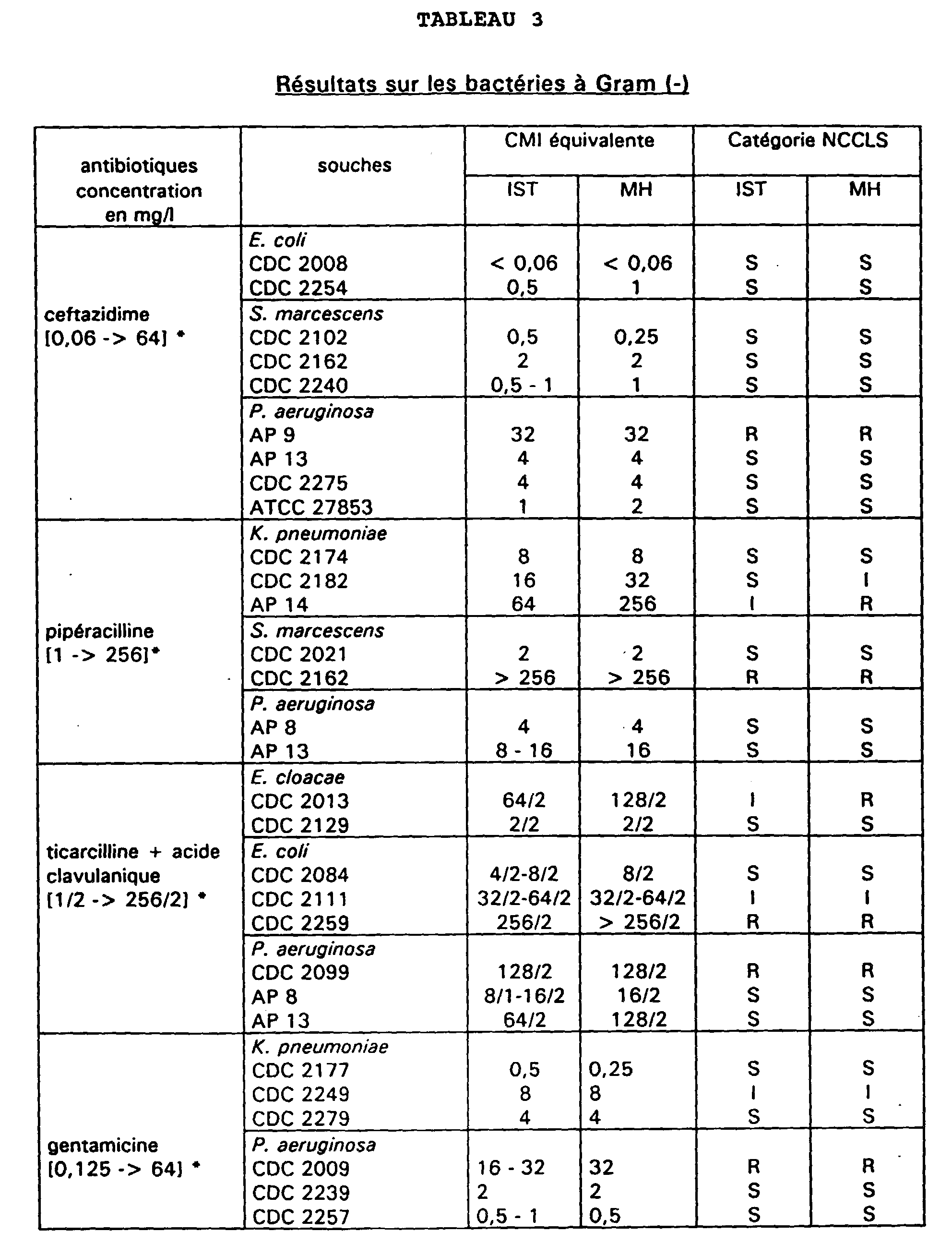

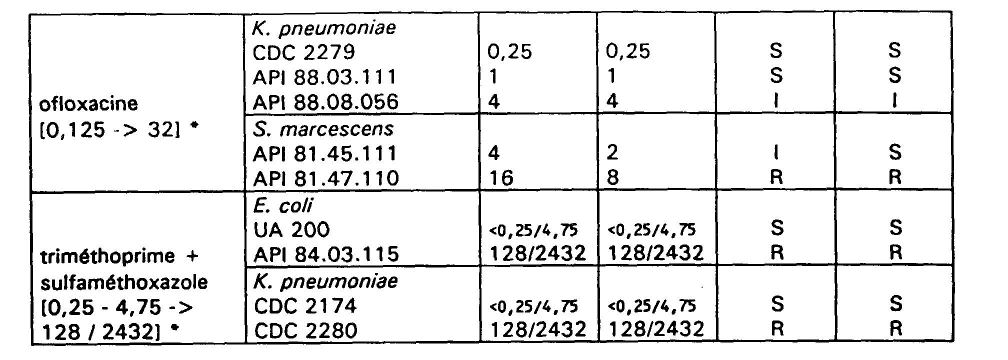

**TABLEAU 4**

| Résultats sur les bactéries à Gram (+) | | | | | |
|---|---|---|---|---|---|
| antibiotiques concentration en mg/l | souches | CMI équivalente | | Catégorie NCCLS | |
| | | IST | MH | IST | MH |
| triméthoprime + sulfaméthoxazole [0,06/1,14 → 32/608] * | *S. aureus* | | | | |
| | ATCC 29213 | < 0,06 | < 0,06 | S | S |
| | CDC 2215 | 0,12 | 0,12 | S | S |
| | CDC 2194 | < 0,06 | < 0,06 | S | S |
| | *S.epidermidis* | | | | |
| | CDC 2024 | < 0,06 | < 0,06 | S | S |
| | CDC 2234 | 16 | 32 | R | R |
| | CDC 2105 | 0,25 | 0,25 | S | S |
| vancomycine [0,5 → 32] * | *S. aureus* | | | | |
| | ATCC 29213 | 2 | 2 | S | S |
| | CDC 2215 | 2 | 1 | S | S |
| | CDC 2194 | 2 | 1 | S | S |
| | *S. epidermidis* | | | | |
| | CDC 2024 | 4 | 4 | S | S |
| | CDC 2234 | 2 | 2 | S | S |
| | CDC 2105 | 4 | 2 | S | S |
| | *E. faecalis* | | | | |
| | *CDC 2017* | 8 | 16 | I | I |
| | *CDC 2230* | 1 | 1-2 | S | S |
| | CDC 2225 | 4 | 4 | S | S |
| érythromicine [0,06 → 32] * | S. aureus | | | | |
| | ATCC 29213 | 0,5 | 0,25 | S | S |
| | CDC 2215 | 0,5 | 0,5 | S | S |
| | CDC 2194 | > 32 | > 32 | R | R |
| | *S. epidermidis* | | | | |
| | CDC 2024 | 0,5 | 0,25 | S | S |
| | CDC 2234 | > 32 | > 32 | R | R |
| | CDC 2105 | 0,25 | 0,25 | S | S |
| | *E. faecalis* | | | | |
| | *CDC 2073* | 16 | 16 | R | R |
| | CDC 2230 | 4 | 4 | I | I |
| oxacilline [0,5 → 16] * | S. aureus | | | | |
| | ATCC 29213 | < 0,5 | < 0,5 | S | S |
| | CDC 2215 | 1 | < 0,5 | S | S |
| | CDC 2194 | 1 | < 0,5 | S | S |
| | *S. epidermidis* | | | | |
| | *CDC 2234* | 2 | 4 | S | R |

| | | | | | |
|---|---|---|---|---|---|
| * Une gamme de concentration, de raison 2, a été testée pour chaque antibiotique dans chacune des fourchettes de concentration précitées. | | | | | |

S, I et R signifient respectivement "sensibles", "intermédiaires" et "résistantes" en référence aux concentrations critiques définies par le NCCLS (National Committee for Clinical Laboratory Standards).
- CDC :: Center Disease Control (Atlanta, Etats-Unis).
- UA et AP :: Collection de souches décrites par Flandrois J.P. et al. "Antibiogramme automatisé" (1988) 1ère édition, Walter Coussement - Bruxelles.
- ATCC :: American Type Culture Collection (USA).
- API :: souchier BIOMERIEUX.

Comme cela ressort des tableaux ci-dessus, il existe une concordance des résultats à plus ou moins une dilution près, ce qui démontre l'équivalence des deux milieux en ce qui concerne l'étude des antibiotiques. Par ailleurs, les résultats montrent que le milieu de l'invention permet de détecter des souches qui présentent un caractère de résistance aux antibiotiques.

### Exemple 4 : comparaison entre le milieu de l'invention et le milieu décrit dans le brevet EP-0 019 054.

Le milieu antérieur a été initialement fabriqué suivant la procédure décrite par Hall (Hall M.J. et al., Roche susceptibility test (RST) medium, a defined formulation for susceptibility testing II, Manufacture stability and use, Journal of Microbiological Method 2 : 215-220 (1984)). Le procédé a cependant dû être légèrement modifié compte tenu de la précipitation de la solution contenant les acides aminés, le magnésium glycéro-phosphate et le calcium gluconate.

Comparaison de la croissance bactérienne :

Les souches respectivement testées sont des entérobactéries, Pseudomonas aeruginosa, des staphylocoques, des entérocoques et des streptocoques de groupe B.

A partir de colonies isolées sur gélose, une suspension a été préparée, ajustée à 0,5 MF dans de l'eau physiologique (densitomètre ATB 1550). La suspension a été diluée au 1/10ème dans de l'eau physiologique, puis rediluée au 1/10ème respectivement dans le milieu RST et le milieu IST de l'invention, préalablement répartis à 1ml par cuvette dans les cassettes de l'appareil MS2 (commercialisé par la société ABBOTT). Les cassettes ont été incubées pendant 10 heures et une lecture a été effectuée toutes les 5 minutes.

Comme illustré à la figure 15, on observe une croissance plus rapide des streptocoques du groupe B avec le milieu de l'invention en comparaison avec le milieu RST, ce qui bien entendu permet une lecture de l'antibiogramme après 5 heures d'incubation par analyse cinétique de la croissance, contre environ 9 heures avec le milieu antérieur.

De même, la cinétique de croissance des entérocoques avec le milieu de l'invention permet de rendre un résultat d'antibiogramme en environ 5 heures, ces mêmes bactéries poussant difficilement avec le milieu de l'art antérieur. Ceci est bien illustré à la figure 16, qui donne une comparaison de la croissance d'une souche d'E. faecium avec les milieux IST et RST.

### LISTE DES SEOUENCES

### 1 INFORMATIONS GENERALES:

1i DEPOSANT:
1iA NOM: BIO MERIEUX
1iB RUE:
1iC VILLE: MARCY L'ETOILE
1iD ETAT OU PROVINCE:
1iE PAYS: FRANCE
1iF CODE POSTAL: 69280

### 1ii TITRE DE L'INVENTION: Milieu nutritif pour la culture de microorganismes

### 1iii NOMBRE DE SEQUENCES: 3

1iv ADRESSE DE CORRESPONDANCE:
1ivA NOM: Cabinet GERMAIN & MAUREAU
1ivB RUE: 20 Bd Eugène Deruelle
1ivC VILLE: LYON
1ivD PAYS: FRANCE
1ivE CODE POSTAL: 69003
1ivF TELEPHONE: 72 60 28 90
1ivG TELEFAX: 78 60 92 85

1v FORME DECHIFFRABLE PAR ORDINATEUR:
1vA TYPE DE SUPPORT: disquette 3,5 pouces DS, HD
1vB ORDINATEUR: EPSON (compatible IBM)
1vC SYSTEME D'EXPLOITATION: DOS
1vD LOGICIEL: MICROSOFT WORD POUR WINDOWS

1vi DEMANDE ACTUELLE:
1viA DATE DE LA DEMANDE: 02/12/1994
1viB NUMERO DE LA DEMANDE: EP 94 420 339.7

1vii DATE DE LA DEMANDE ANTERIEURE: 02/12/1993
1viiA NUMERO DE LA DEMANDE: 93 14687
1viiB CLASSIFICATION:

1viii MANDATAIRE:
1viiiA NOM: CABINET GERMAIN & MAUREAU, Dominique GUERRE
1viiiB REFERENCE DOSSIER: MD/B 05 B 1878 EP

2 INFORMATIONS POUR LA SEQ ID NO: 1
2i CARACTERISTIQUES DE LA SEQUENCE:
2iA LONGUEUR: 4 acides aminés
2iB TYPE: séquence d'acides aminés

### 2ii TYPE DE MOLECULE: peptide

2vi ORIGINE:
2viA ORGANISME:
2viB SOUCHE:

2vii SOURCE IMMEDIATE: bioMérieux S.A.
2viiA BIBLIOTHEQUE:
2xi DESCRIPTION DE LA SEQUENCE : SEQ ID NO: 1 
Ala Gly Ser Glu
1
2 INFORMATIONS POUR LA SEQ ID NO: 2
2i CARACTERISTIQUES DE LA SEQUENCE:
2iA LONGUEUR: 12 acides aminés
2iB TYPE: séquence d'acides aminés

### 2ii TYPE DE MOLECULE: peptide

2vi ORIGINE:
2viA ORGANISME:
2viB SOUCHE:

2vii SOURCE IMMEDIATE: bioMérieux S.A.
2viiA BIBLIOTHEQUE:
2xi DESCRIPTION DE LA SEQUENCE : SEQ ID NO: 2 
Glu Asp Arg Pro Pro Leu Phe Gly Gln Gly Thr Val
1               5                   10
2 INFORMATIONS POUR LA SEQ ID NO: 3
2i CARACTERISTIQUES DE LA SEQUENCE:
2iA LONGUEUR: 11 acides aminés
2iB TYPE: séquence d'acides aminés

### 2ii TYPE DE MOLECULE: peptide

2vi ORIGINE:
2viA ORGANISME:
2viB SOUCHE:

2vii SOURCE IMMEDIATE: bioMérieux S.A.
2viiA BIBLIOTHEQUE:
2xi DESCRIPTION DE LA SEQUENCE : SEQ ID NO: 3

Ala Ser Asp Ala Lys Ala Tyr Asp Thr Glu Val
1               5                   10

## Revendications

1. Milieu nutritif pour la culture de microorganismes, caractérisé en ce qu'il comprend 7-70 mg/l de L-alanine, 8-80 mg/l de L-arginine, 5-50 mg/l de L-asparagine, 6-60 mg/l d'acide L-aspartique, 2-20 mg/l de L-cystéine, 4-40 mg/l de L-glutamine, 8-80 mg/l d'acide L-glutamique, 7-70 mg/l de glycine, 2,5-25 mg/l de L-histidine, 6-60 mg/l de L-isoleucine, 9-90 mg/l de L-leucine, 9-90 mg/l de L-lysine, 4-40 mg/l de L-méthionine, 5-50 mg/l de L-phénylalanine, 4-40 mg/1 de L-proline, 4-400 mg/l de L-sérine, 5-50 mg/l de L-thréonine, 2-20 mg/l de L-tryptophane, 4-40 mg/l de L-tyrosine, 8-80 mg/l de L-valine, 0,01-0,2 mg/l de biotine, 0,01-0,3 mg/l de pantothénate de calcium, 0,001-0,01 mg/l d'acide folique, 0,01-0,5 mg/l d'inositol, 0,04-0,2 mg/l de vitamine PP, 0,02-0,3 mg/l de vitamine B6, 0,01-0,1 mg/l de chlorure de thiamine, 0,005-0,05 mg/l d'acide lipoïque, 0,1-5 mg/l de choline, 1-10 mg/l d'oxaloacétate d'éthyle, 0,1-1 mg/l de spermidine, 5-50 mg/l de Tween® 80, 11-110 mg/l de nucléosides puriques consistant en l'adénosine et la guanosine, dans un rapport de la première à la seconde compris entre environ 0,8 et 1,2, et 4-40 mg/l de nucléosides pyrimidiques, consistant en la cytidine et l'uridine, dans un rapport de la première à la seconde compris entre 1 et 3, les nucléosides puriques et les nucléosides pyrimidiques étant présents dans un rapport des premiers aux seconds compris entre 1,5 et 2,2, 100-10000 mg/l de glucose, 100-10000 mg/l d'acide malique, 0,020-0,583 mg/l de fer, 49,3-493 mg/l de potassium, 1,2-12 mg/l de magnésium, 2,7-27,2 mg/l de calcium, 1,18-3,15 g/l de sodium, 1820-5000 mg/l en chlorure, 17,8-178,3 mg/l en ion phosphate, 33,6-336,0 mg/l en ion ammonium, 2000-20000 mg/l d'un tampon acide, et 1000-10000 mg/l d'un tampon basique.

2. Milieu selon la revendication 1, caractérisé en ce qu'il comprend 5-50 mg/l d'adénosine, 3-30 mg/l de cytidine, 6-60 mg/l de guanosine, 1-10 mg/l d'uridine, 0,2-5 mg/l de pyrophosphate ferrique, 100-1000 mg/l dephosphate de potassium dibasique, 10-110 mg/l de chlorure de magnésium, 10-100 mg/l de chlorure de calcium, 100-1000 mg/l de chlorure d'ammonium, 3000-8000 mg/l de chlorure de sodium.

3. Milieu selon 1 ou 2 caractérisé en ce qu'il comprend en outre jusqu'à 0,022 mg/l de molybdène, jusqu'à 0,010 mg/l de cobalt, jusqu'à 0,027 mg/l de manganèse, jusqu'à 0,007 mg/l de zinc, jusqu'à 0,128 mg/l de cuivre, jusqu'à 0,088 mg/l de bore, jusqu'à 18,4 mg/l d'ion sulfate, jusqu'à 0,1 mg/l de riboflavine, jusqu'à 0,005 mg/l de cyanocobalamine, jusqu'à 0,1 mg/l de ménadione, jusqu'à 0,5 mg/l d'acide borique et/ou jusqu'à 5 mg/l de glycérol.

4. Milieu selon la revendication 3, caractérisé en ce qu'il comprend jusqu'à 0,04 mg/l de molybdate d'ammonium, jusqu'à 0,04 mg/l de chlorure de cobalt, jusqu'à 0,1 mg/l de chlorure de manganèse, jusqu'à 0,03 mg/l de sulfate de zinc, jusqu'à 0,5 mg/l de sulfate de cuivre, et/ou jusqu'à 100 mg/l de sulfate de potassium.

5. Milieu selon l'une ou l'autre des revendications 1 et 2, caractérisé en ce que (a) le tampon acide est le tampon MOPS, (b) le tampon basique est le tampon Tris, et (c) il comprend en outre 0,0005-0,005 mg/l de cyanocobalamine, 0,01-0,1 mg/l de ménadione, 0,01-0,1 mg/l de riboflavine, 0,1-5 mg/l de glycérol, 0,0004-0,04 mg/l de molybdate d'ammonium, 0,0004-0,04 mg/l de chlorure de cobalt, 0,001-0,1 mg/l de chlorure de manganèse, et 0,0003-0,03 mg/l de sulfate de zinc.

6. Milieu selon la revendication 1, caractérisé en ce qu'il comprend au moins 65 mg/l d'alanine, 73 mg/l de L-arginine, 43 mg/l de L-asparagine, 53 mg/l de L-acide aspartique, 16 mg/l de L-cystéine, 37 mg/l de L-glutamine, 73 mg/l de L-acide glutamique, 66 mg/l de glycine, 22 mg/l de L-histidine, 54 mg/l de L-isoleucine, 84 mg/l de L-leucine, 89 mg/l de L-lysine, 33 mg/l de L-méthionine, 44 mg/l de L-phénylalanine, 36 mg/l de L-proline, 320 mg/l de L-sérine, 43 mg/l de L-thréonine, 17 mg/l de L-tryptophane, 36 mg/l de L-tyrosine, 70 mg/l de L-valine, 0,03 mg/l de biotine, 0,03 mg/l de pantothénate de calcium, 0,005 mg/l d'acide folique, 0,15 mg/l d'inositol, 0,15 mg/l de vitamine PP, 0,15 mg/l de vitamine B6, 0,03 mg/l de riboflavine, 0,03 mg/l de chlorure de thiamine, 0,01 mg/l d'acide lipoïque, 1,5 mg/l de choline, 2,5 mg/l de glycérol, 5 mg/l d'oxaloacétate d'éthyle, 0,75 mg/l de spermidine, 10 mg/l de Tween® 80, 15 mg/l d'adénosine, 10 mg/l de cytidine, 20 mg/l de guanosine, 6 mg/l d'uridine, 750 mg/l de glucose, 500 mg/l d'acide malique, 0,004 mg/l de molybdate d'ammonium, 0,004 mg/l de chlorure de cobalt, 0,016 mg/l de chlorure de manganèse, 0,003 mg/l de sulfate de zinc, 1,8 mg/l de pyrophosphate ferrique, 0,25 mg/l de sulfate de cuivre, 0,025 mg/l d'acide borique, 50 mg/l de sulfate de potassium, 230 mg/l de phosphate de potassium dibasique, 100 mg/l de chlorure de magnésium, 90 mg/l de chlorure de calcium, 500 mg/l de chlorure d'ammonium, 7500 mg/l de chlorure de sodium, 15000 mg/l de tampon MOPS, 7000 mg/l de tampon Tris, et en ce qu'il comprend en outre 0,0008 mg/l de cyanocobalamine et 0,05 mg/l de ménadione.

7. Milieu selon l'une des revendications précédentes, caractérisé en ce qu'il comprend de plus au moins un peptide à une concentration comprise entre 50 et 100 mg/l ou un mélange de peptides à une concentration comprise entre 50 et 1000 mg/l, lesdits peptides présentant une longueur comprise entre 4 et 15 acides aminés.

8. Milieu selon la revendication 7, caractérisé en ce que le peptide est choisi parmi les peptides présentant les séquences d'acides aminés suivantes: Ala-Gly-Ser-Glu (SEQ ID NO: 1), Glu-Asp-Arg-Pro-Pro-Leu-Phe-Gly-Gln-Gly-Thr-Val (SEQ ID NO: 2), et

Ala-Ser-Asp-Ala-Lys-Ala-Tyr-Asp-Thr-Glu-Val (SEQ ID NO: 3).

9. Milieu selon l'une des revendications précédentes, caractérisé en ce qu'il comprend en outre un extrait de boeuf, en une concentration préférentiellement ccmprise entre 2 et 10 g/l.

10. Milieu selon l'une des revendications précédentes, caractérisé en ce qu'il comprend un agent gélifiant, en particulier de l'agarose, à une concentration d'environ 10000 mg/l.

11. Utilisation du milieu tel que défini dans l'une quelconque des revendications précédentes dans la détermination de l'activité d'une substance thérapeutiquement active contre un microorganisme infectieux.

## Patentansprüche

1. Nährmedium für die Kultivierung von Mikroorganismen, dadurch gekennzeichnet, daß es folgendes enthält, nämlich 7-70 mg/l L-Alanin, 8-80 mg/l L-Arginin, 5-50 mg/l L-Asparagin, 6-60 mg/l L-Asparaginsäure, 2-20 mg/l L-Cystein, 4-40 mg/l L-Glutamin, 8-80 mg/l L-Glutaminsäure, 7-70 mg/l Glycin, 2,5-25 mg/l L-Histidin, 6-60 mg/l L-Isoleucin, 9-90 mg/l L-Leucin, 9-90 mg/l L-Lysin, 4-40 mg/l L-Methionin, 5-50 mg/l L-Phenylalanin, 4-40 mg/l L-Prolin, 4-400 mg/l L-Serin, 5-50 mg/l L-Threonin, 2-20 mg/l L-Tryptophan, 4-40 mg/l L-Tyrosin, 8-80 mg/l L-Valin, 0,01-02 mg/l Biotin, 0,01-0,3 mg/l Calcium-pantothenat, 0,001-0,01 mg/l Folsäure, 0,01-0,5 mg/l Inositol, 0,04-0,2 mg/l Vitamin PP, 0,02-0,3 mg/l Vitamin B₆, 0,01-0,1 mg/l Thiaminchlorid, 0,005-0,05 mg/l Liponsäure, 0,1-5 mg/l Cholin, 1-10 mg/l Ethyloxalacetat, 0,1-1 mg/l Spermidin, 5-50 mg/l Tween® 80, 11-110 mg/l Purin-Nucleoside bestehend aus Adenosin und Guanosin, die in einem Verhältnis vom ersten zum zweiten zwischen 0,8 und 1,2 enthalten sind, und 4-40 mg/l Pyrimidin-Nucleoside bestehend aus Cytidin und Uridin, in einem Verhältnis vom ersten zum zweiten zwischen 1 und 3 enthalten sind, wobei die Purin-Nucleoside und die Pyrimidin-Nucleoside in einem Verhältnis von den ersten zu den zweiten zwischen 1,5 und 2,2 vorhanden sind, 100-10000 mg/l Glucose, 100-10000 mg/l Maleinsäure, 0,020-0,583 mg/l Eisen, 49,3-493 mg/l Kalium, 1,2-12 mg/l Magnesium, 2,7-27,2 mg/l Calcium, 1,18-3,15 g/l Natrium, 1820-5000 mg/l Chlor, 17,8-178,3 mg/l Phosphationen, 33,6-336,0 mg/l Ammoniumionen, 2000-20000 mg/l eines sauren Puffers und 1000-10000 mg/l eines basischen Puffers.

2. Medium nach Anspruch 1, dadurch gekennzeichnet, daß es folgendes enthält, nämlich 5-50 mg/l Adenosin, 3-30 mg/l Cytidin, 6-60 mg/l Guanosin, 1-10 mg/l Uridin, 0,2-5 mg/1 Eisenpyrophosphat, 100-1000 mg/l zweibasiges Kaliumphosphat, 10-110 mg/l Magnesiumchlorid, 10-100 mg/l Calciumchlorid, 100-1000 mg/l Ammoniumchlorid, 3000-8000 mg/l Natriumchlorid.

3. Medium nach Anspruch 1 oder 2, dadurch gekennzeichnet, daß es darüber hinaus folgendes enthält, nämlich bis zu 0,022 mg/l Molybdän, bis zu 0,010 mg/l Cobalt, bis zu 0,027 mg/l Mangan, bis zu 0,007 mg/l Zink, bis zu 0,128 mg/l Kupfer, bis zu 0,088 mg/l Bor, bis zu 18,4 mg/l Sulfationen, bis zu 0,1 mg/l Riboflavin, bis zu 0,005 mg/l Cyanocobalamin, bis zu 0,1 mg/l Menadion, bis zu 0,5 mg/l Borsäure und/oder bis zu 5 mg/l Glycerin.

4. Medium nach Anspruch 3, dadurch gekennzeichnet, daß es bis zu 0,04 mg/l Ammoniummolybdat, bis zu 0,04 mg/l Cobaltchlorid, bis zu 0,1 mg/l Manganchlorid, bis zu 0,03 mg/l Zinksulfat, bis zu 0,5 mg/l Kupfersulfat und/oder bis zu 100 mg/l Kaliumsulfat enthält.

5. Medium nach einem der Ansprüche 1 oder 2, dadurch gekennzeichnet, daß (a) der saure Puffer der Puffer MOPS ist, und (b) der basische Puffer der Puffer Tris ist, und (c) daß es darüber hinaus 0,0005-0,005 mg/l Cyanocobalamin, 0,01-0,1 mg/l Menadion, 0,01-0,1 mg/l Riboflavin, 0,1-5 mg/l Glycerin, 0,0004-0,04 mg/l Ammoniummolybdat, 0,0004-0,04 mg/l Cobaltchlorid, 0,001-0,1 mg/l Manganchlorid und 0,0003-0,03 mg/l Zinksulfat enthält.

6. Medium nach Anspruch 1, dadurch gekennzeichnet, daß es zumindest 65 mg/l Alanin, 73 mg/l L-Arginin, 43 mg/l L-Aspargin, 53 mg/l L-Asparaginsäure, 16 mg/l L-Cystein, 37 mg/l L-Glutamin, 73 mg/l L-Glutaminsäure, 66 mg/l Glycin, 22 mg/l L-Histidin, 54 mg/l L-Isoleucin, 84 mg/l L-Leucin, 89 mg/l L-Lysin, 33 mg/l L-Methionin, 44 mg/l L-Phenylalanin, 36 mg/l L-Prolin, 320 mg/l L-Serin, 43 mg/l L-Threonin, 17 mg/l L-Tryptophan, 36 mg/l L-Tyrosin, 70 mg/l L-Valin, 0,03 mg/l Biotin, 0,03 mg/l Calciumpantothenat, 0,005 mg/l Folsäure, 0,15 mg/l Inositol, 0,15 mg/l Vitamin PP, 0,15 mg/l Vitamin B₆, 0,03 mg/l Riboflavin, 0,03 mg/l Thiaminchlorid, 0,01 mg/l Liponsäure, 1,5 mg/l Cholin, 2,5 mg/l Glycerin, 5 mg/l Ethyloxalacetat, 0,75 mg/l Spermidin, 10 mg/l Tveen® 80, 15 mg/l Adenosin, 10 mg/l Cytidin, 20 mg/l Guanosin, 6 mg/l Uridin, 750 mg/l Glucose, 500 mg/l Maleinsäure, 0,004 mg/l Ammoniummolybdat, 0,004 mg/l Cobaltchlorid, 0,016 mg/l Manganchlorid, 0,003 mg/l Zinksulfat, 1,8 mg/l Eisenpyrophosphat, 0,25 mg/l Kupfersulfat, 0,025 mg/l Borsäure, 50 mg/l Kaliumsulfat, 230 mg/l zweibasisches Kaliumphosphat, 100 mg/l Magnesiumchlorid, 90 mg/l Calciumchlorid, 500 mg/l Ammoniumchlorid, 7500 mg/l Natriumchlorid, 15000 mg/l MOPS-Puffer, 7000 mg/l Tris-Puffer enthält, und daß es darüber hinaus 0,0008 mg/l Cyanocobalamin und 0,05 mg/l Menadion enthält.

7. Medium nach einem der vorhergehenden Ansprüche, dadurch gekennzeichnet, daß es zumindest ein Peptid in einer Konzentration zwischen 50 und 100 mg/l oder eine Mischung von Peptiden in einer Konzentration zwischen 50 und 1000 mg/l enthält, wobei die genannten Peptide eine Länge zwischen 4 und 15 Aminosäuren aufweisen.

8. Medium nach Anspruch 7, dadurch gekennzeichnet, daß das Peptid ausgewählt ist aus den Peptiden, die die folgenden Aminosäuresequenzen aufweisen: 
Ala-Gly-Ser-Glu (SEQ ID NO: 1)
Glu-Asp-Arg-Pro-Pro-Leu-Phe-Gly-Gln-Gly-Thr-Val
(SEQ ID NO: 2)
 und

Ala-Ser-Asp-Ala-Lys-Ala-Tyr-Asp-Thr-Glu-Val
(SEQ ID NO: 3).

9. Medium nach einem der vorhergehenden Ansprüche, dadurch gekennzeichnet, daß es darüber hinaus einen Rinderextrakt in einer Konzentration von vorzugsweise zwischen 2 und 10 g/l enthält.

10. Medium nach einem der vorhergehenden Ansprüche, dadurch gekennzeichnet, daß es ein gelbildendes Agens, insbesondere Agarose enthält, und zwar in einer Konzentration von etwa 10000 mg/l.

11. Verwendung des Mediums nach einem der vorhergehenden Ansprüche zur Bestimmung der Aktivität eines therapeutischen Wirkstoffes, der gegen einen infektiösen Mikroorganismus aktiv ist.

## Claims

1. Nutritive medium for the culture of microorganisms, characterized in that it contains 7-70 mg/l of L-alanine, 8-80 mg/l of L-arginine, 5-50 mg/l of L-asparagine, 6-60 mg/l of L-aspartic acid, 2-20 mg/l of L-cysteine, 4-40 mg/l of L-glutamine, 8-80 mg/l of L-glutamic acid, 7-70 mg/l of glycine, 2.5-25 mg/l of L-histidine, 6-60 mg/l of L-isoleucine, 9-90 mg/l of L-leucine, 9-90 mg/l of L-lysine, 4-40 mg/l of L-methionine, 5-50 mg/l of L-phenylalanine, 4-40 mg/l of L-proline, 4-400 mg/l of L-serine, 5-50 mg/l of L-threonine, 2-20 mg/l of L-tryptophan, 4-40 mg/l of L-tyrosine, 8-80 mg/l of L-valine, 0.01-0.2 mg/l of biotin, 0.01-0.3 mg/l of calcium pantothenate, 0.001-0.01 mg/l of folic acid, 0.01-0.5 mg/l of inositol, 0.04-0.2 mg/l of nicotinamide, 0.02-0.3 mg/l of vitamin B6, 0.01-0.1 mg/l of thiamine chloride, 0.005-0.05 mg/l of lipoic acid, 0.1-5 mg/l of choline, 1-10 mg/l of ethyl oxaloacetate, 0.1-1 mg/l of spermidine, 5-50 mg/l of Tween® 80, 11-110 mg/l of purine nucleosides consisting of adenosine and guanosine, in a ratio of the first to the second of between approximately 0.8 and 1.2, and 4-40 mg/l of pyrimidine nucleosides consisting of cytidine and uridine, in a ratio of the first to the second of between 1 and 3, the purine nucleosides and the pyrimidine nucleosides being present in a ratio of the first to the second of between 1.5 and 2.2, 100-10 000 mg/l of glucose, 100-10 000 mg/l of malic acid, 0.020-0.583 mg/l of iron, 49.3-493 mg/l of potassium, 1.2-12 mg/l of magnesium, 2.7-27.2 mg/l of calcium, 1.18-3.15 g/l of sodium, 1 820-5 000 mg/l of chloride, 17.8-178.3 mg/l of phosphate ion, 33.6-336.0 mg/l of ammonium ion, 2 000-20 000 mg/l of an acidic buffer and 1 000-10 000 mg/l of a basic buffer.

2. Medium according to Claim 1, characterized in that it contains 5-50 mg/l of adenosine, 3-30 mg/l of cytidine, 6-60 mg/l of guanosine, 1-10 mg/l of uridine, 0.2-5 mg/l of ferric pyrophosphate, 100-1 000 mg/l of dibasic potassium phosphate, 10-110 mg/l of magnesium chloride, 10-100 mg/l of calcium chloride, 100-1 000 mg/l of ammonium chloride, 3 000-8 000 mg/l of sodium chloride.

3. Medium according to claim 1 or 2, characterized in that it contains, in addition, up to 0.022 mg/l of molybdenum, up to 0.010 mg/l of cobalt, up to 0.027 mg/l of manganese, up to 0.007 mg/l of zinc, up to 0.128 mg/l of copper, up to 0.088 mg/l of boron, up to 18.4 mg/l of sulphate ion, up to 0.1 mg/l of riboflavin, up to 0.005 mg/l of cyanocobalamin, up to 0.1 mg/l of menadione, up to 0.5 mg/l of boric acid and/or up to 5 mg/l of glycerol.

4. Medium according to Claim 3, characterized in that it contains up to 0.04 mg/l of ammonium molybdate, up to 0.04 mg/l of cobalt chloride, up to 0.1 mg/l of manganese chloride, up to 0.03 mg/l of zinc sulphate, up to 0.5 mg/l of copper sulphate and/or up to 100 mg/l of potassium sulphate.

5. Medium according to either one of Claims 1 and 2, characterized in that (a) the acidic buffer is MOPS buffer, (b) the basic buffer is Tris buffer, and (c) it contains, in addition, 0.0005-0.005 mg/l of cyanocobalamin, 0.01-0.1 mg/l of menadione, 0.01-0.1 mg/l of riboflavin, 0.1-5 mg/l of glycerol, 0.0004-0.04 mg/l of ammonium molybdate, 0.0004-0.04 mg/l of cobalt chloride, 0.001-0.1 mg/l of manganese chloride and 0.0003-0.03 mg/l of zinc sulphate.

6. Medium according to Claim 1, characterized in that it contains at least 65 mg/l of alanine, 73 mg/l of L-arginine, 43 mg/l of L-asparagine, 53 mg/l of L-aspartic acid 16 mg/l of L-cysteine, 37 mg/l of L-glutamine, 73 mg/l of L-glutamic acid , 66 mg/l of glycine, 22 mg/l of L-histidine, 54 mg/l of L-isoleucine, 84 mg/l of L-leucine, 89 mg/l of L-lysine, 33 mg/l of L-methionine, 44 mg/l of L-phenylalanine, 36 mg/l of L-proline, 320 mg/l of L-serine, 43 mg/l of L-threonine, 17 mg/l of L-tryptophan, 36 mg/l of L-tyrosine, 70 mg/l of L-valine, 0.03 mg/l of biotin, 0.03 mg/l of calcium pantothenate, 0.005 mg/l of folic acid, 0.15 mg/l of inositol, 0.15 mg/l of nicotinamide, 0.15 mg/l of vitamin B6, 0.03 mg/l of riboflavin, 0.03 mg/l of thiamine chloride, 0.01 mg/l of lipoic acid, 1.5 mg/l of choline, 2.5 mg/l of glycerol, 5 mg/l of ethyl oxaloacetate, 0.75 mg/l of spermidine, 10 mg/l of Tween® 80, 15 mg/l of adenosine, 10 mg/l of cytidine, 20 mg/l of guanosine, 6 mg/l of uridine, 750 mg/l of glucose, 500 mg/l of malic acid, 0.004 mg/l of ammonium molybdate, 0.004 mg/l of cobalt chloride, 0.016 mg/l of manganese chloride, 0.003 mg/l of zinc sulphate, 1.8 mg/l of ferric pyrophosphate, 0.25 mg/l of copper sulphate, 0.025 mg/l of boric acid, 50 mg/l of potassium sulphate, 230 mg/l of dibasic potassium phosphate, 100 mg/l of magnesium chloride, 90 mg/l of calcium chloride, 500 mg/l of ammonium chloride, 7,500 mg/l of sodium chloride, 15 000 mg/l of MOPS buffer, 7 000 mg/l of Tris buffer, and in that it contains, in addition, 0.0008 mg/l of cyanocobalimin and 0.05 mg/l of menadione.

7. Medium according to one of the preceding claims, characterized in that it additionally contains at least one peptide at a concentration of between 50 and 100 mg/l or a mixture of peptides at a concentration of between 50 and 1 000 mg/l, the said peptides having a length of between 4 and 15 amino acids.

8. Medium according to Claim 7, characterized in that the peptide is chosen from amongst the peptides having the following amino acid sequences: 
Ala-Gly-Ser-Glu (SEQ ID NO:1),
Glu-Asp-Arg-Pro-Pro-Leu-Phe-Gly-Gln-Gly-Thr-Val
(SEQ ID NO:2)
 and

Ala-Ser-Asp-Ala-Lys-Ala-Tyr-Asp-Thr-Glu-Val (SEQ
ID NO:3).

9. Medium according to one of the preceding claims, characterized in that it contains in addition a beef extract, preferably in a concentration of between 2 and lo g/l.

10. Medium according to one of the preceding claims, characterized in that it contains a gelling agent, in particular agarose, at a concentration of approximately 10 000 mg/l.

11. Use of the medium such as defined in any one of the preceding claims in the determination of the activity of a substance which is therapeutically active against an infectious microorganism.
